(19) Europäisches Patentamt
European Patent Office
Office européen des brevets

(11) Veröffentlichungsnummer: **0 006 454**
**A1**

(12) **EUROPÄISCHE PATENTANMELDUNG**

(21) Anmeldenummer: **79101620.7**

(22) Anmeldetag: **28.05.79**

(51) Int. Cl.³: **C 07 C 87/14,** C 07 C 85/06, C 07 C 93/04

(30) Priorität: **07.06.78 DE 2824908**

(43) Veröffentlichungstag der Anmeldung: **09.01.80 Patentblatt 80/1**

(84) Benannte Vertragsstaaten: **BE CH DE FR IT NL**

(71) Anmelder: **BASF Aktiengesellschaft, Carl-Bosch-Strasse 38, D-6700 Ludwigshafen (DE)**

(72) Erfinder: **Mesch, Walter, Dr. Dipl.-Chem., Richinesstrasse 11, D-6700 Ludwigshafen 29 (DE)**

(54) **Verfahren zur Herstellung von w,w'-Diaminoalkanen, -oxaalkanen oder -azaalkanen.**

(57) Amine der Formel I

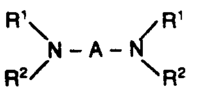

I,

in der A einen gegebenenfalls durch Sauerstoff- oder Stickstoffatome unterbrochenen Alkylenrest mit 4 bis 10 Kohlenstoffatomen und $R^1$ und $R^2$ Alkylreste, die hydrierbeständige Substituenten tragen können, bedeuten oder die zusammen einen heterocyclischen 5- oder 6-gliedrigen Ring bilden werden durch Umsetzung eines Diols der Formel II

$$HOCH_2 - A - CH_2OH \qquad II,$$

mit einem Amin

$R^1$ $R^2$ NH, wobei A, $R^1$ und $R^2$ die oben angegebenen Bedeutungen hat, in Gegenwart von Wasserstoff in flüssiger Phase erhalten, indem man die Umsetzung mit einem molaren Überschuß des Amins an einem Kupferchromitkatalysator bei einem Wasserstoffdruck über 5 bar und Temperaturen von 150 bis 250° C kontinuierlich vornimmt.

EP 0 006 454 A1

BEZEICHNUNG GEÄNDERT
siehe Titelseite



Verfahren zur Herstellung von ω,ω'-Bis-dialkylaminoalkanen

Die Erfindung betrifft die Herstellung von ω,ω'-Bis-dialkylaminoalkanen durch katalytische Umsetzung von ω,ω'-Alkandiolen mit Dialkylaminen unter Wasserstoffdruck.

Ditertiäre Amine sind wertvolle Agentien als Chelatbildner von Metallhydriden, Säurefängern und finden Verwendung als aprotische Lösungsmittel, als Katalysatoren, als Bestandteile von fotographischen Entwicklern und von Raketentreibstoffen.

Die bisher bekannten Herstellungsmethoden ditertiärer Amine befriedigen nicht, da sie für die großtechnische Herstellung wenig geeignet sind.

Dazu zählen die Methoden der Umsetzung der entsprechenden ω,ω'-Dichlorverbindungen mit Dialkylaminen, der Alkylierung der ω,ω'-Diamine, der katalytischen Hydrierung der Bis-dialkylaminoalkene und -alkyne, des thermischen Abbaues der quartären Alkylammoniumhydroxide und der elektrolytischen Reduktion der Tetraalkylbiscarbonamide mit $Li\ Al\ H_4$.

Mu/ML

Auch die Herstellung solcher ditertiärer Amine durch aminierende Hydrierung ist schon aus der US-PS 3 223 734 bekannt. Jedoch besteht der wesentliche Unterschied der dort beschriebenen Arbeitsweise gegenüber der Erfindung darin, daß ohne Wasserstoff und mit Raney-Nickel als Katalysator praktisch ohne erhöhten Druck gearbeitet wird. Hierdurch entstehen erhebliche Mengen unerwünschter Kondensations- und Nebenprodukte, wodurch die Ausbeute abfällt. In den Beispielen 17 und 18 wird Dodecanol und hydrierter Talgalkohol in Gegenwart von Raney-Nickel mit großen Überschüssen von Dimethylamin bzw. Methylamin zum tertiären Dimethyldodecylamin (69,5 %) bzw. zum tertiären Di-(talgalkyl)-methylamin (79,1 %) umgesetzt. Wie die Autoren der DE-OS 25 35 073 festgestellt haben, hat sich durch Nacharbeitung der Beispiele ergeben, daß die beiden genannten tertiären Amine noch erhebliche Mengen anderer tertiärer Amine enthalten. Dies soll darauf beruhen, daß die eingesetzten Dimethyl- und Methylamine in Gegenwart von Raney-Nickel u.a. in Monomethyl- bzw. Dimethylamine disproportionieren, wodurch dann andere tertiäre Amine als erwartet entstehen und wodurch die Ausbeute der ausgewiesenen tertiären Amine überhöht erscheint.

Für eine großtechnische Herstellung von beispielsweise langkettigen tertiären Dimethylalkylaminen bzw. tertiären Methyldialkylaminen ist das Verfahren der US-PS 3 223 734, abgesehen von den geringen Ausbeuten, somit deshalb nicht geeignet, weil die erzeugten tertiären Amine nicht die erforderliche Reinheit aufweisen. Für bifunktionelle Amine gilt dies in erhöhtem Maß.

Der Vorschlag der DE-OS 25 35 073 wiederum ist auf die Mitverwendung relativ großer Wasserstoffmengen angewiesen, die eine ständige Umwälzung großer Gasmengen erfordert, zumal auch die Aminkomponente der Reaktionsteilnehmer in Gasform angewendet wird.

Es bestand daher die Aufgabe ein Verfahren vorzuschlagen, bei dem man von leicht zugänglichen Materialien ausgehen und gleichzeitig auf technisch einfachem Wege wirtschaftliche Ausbeuten erzielen kann.

Den Vorteil guter Zugänglichkeit haben in diesem Sinne z.B. die ω,ω'-Diole Butandiol-1.4, Pentandiol-1.5, Hexandiol-1.6. Besonders gut verfügbar ist das Gemisch der drei Diole aus der Hydrierung von Rückstandsdicarbonsäuren der Oxidation von Cyclohexanol zu Adipinsäure.

Auch ist der Austausch der alkoholischen Gruppe gegen die Amino-, Monoalkylamino- und Dialkylaminogruppierungen eine eingehend untersuchte Reaktion (vgl. Houben-Weyl, Methoden der organischen Chemie, Georg Thieme Verlag Stuttgart, 4. Aufl., 11/1, Seiten 108 ff und Spialter und Pappalardo, The Acytic, Aliphatic Tertiary Amines, New York, The MacMillan Company 1965, Seite 35). Es fällt jedoch auf, daß die erzielten Ausbeuten in der zumeist diskontinuierlich durchgeführten Umsetzung mäßig sind. Dies wird u.a. auf die besonders bei primären Alkoholen auftretende Aldolkondensation und die Bildung von Azomethin zurückgeführt.

In der ausführlichen Übersicht zur katalytischen Aminierung langkettiger, aliphatischer Alkohole (A. Baiker und W. Richarz, Ind. Engl. Chem., Prod. Res. Deu., 1977, Vol 16, Seiten 261 bis 266) wird Kupferchromit als Katalysator angegeben; die beste Umsetzungstemperatur soll bei 300°C liegen; die Ausbeute fällt jedoch schon bei 250°C von 98 % auf 80 % und bei 230°C auf 50 % ab. Das Molverhältnis Dimethylamin/Alkohol im Reaktionsgemisch ist dabei 1,5:1 (Bild 1, l.c.).

Bild 2 dieser Veröffentlichung zeigt weiterhin die Abhängigkeit der Umsetzung von diesem Molverhältnis der Reaktanden: sie erhöht sich mit steigender und sinkt mit abnehmender Konzentration an Dimethylamin. Bei kontinuierlicher Durchführung der Umsetzung ist sogar ein 5,5 molarer Dimethylaminüberschuß gegenüber dem Alkohol für die optimale Umsetzung nötig.

Der Vergleich der angeführten Verfahren zeigt die bisher technisch wenig befriedigende Art der Umsetzung von Monoalkoholen mit Aminen:

Die Anhebung von Umsatz und Ausbeute durch Ausschleusen von Reaktionswasser mit einer hohen Menge Kreisgas erfordert zu dessen Abscheidung kostspielige Kühlfläche.

Die Aufgabe der Herstellung von ω,ω'-Bis-dimethylaminoalkanen oder -oxoalkanen aus den entsprechenden ω,ω'-Diolen, noch dazu in kontinuierlicher Weise ist nach den angegebenen Verfahrensarten für Monoalkohole technisch kaum lösbar: die Abscheidung der erhöhten Wasseranteile aus der hohen Menge Kreisgas wird zusätzlich noch erschwert durch die Gegenwart der wasserlöslichen Bis-dialkylaminoalkane und ihrer Zwischenstufen; die gemäß dem Stand der Technik zur Umsetzung nötigen Temperaturen über 250°C liefern viel Rückstand.

Es wurde nun überraschenderweise gefunden, daß man ω,ω'-Bis-dialkylaminoalkane der Formel I

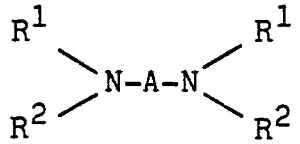

I,

in der A einen gegebenenfalls durch Sauerstoff- oder Stickstoffatome unterbrochenen Alkylenrest mit 4 bis 10 Kohlenstoffatomen und $R^1$ und $R^2$ Alkylreste, die hydrierbeständige Substituenten tragen können, bedeuten, oder die zusammen einen heterocyclischen 5- oder 6-gliedrigen Ring bilden, durch Umsetzung eines Diols der Formel II

$$HOCH_2\text{-}A\text{-}CH_2OH \qquad \text{II,}$$

mit einem Amin 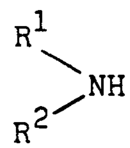, wobei A, $R^1$ und $R^2$ die oben angegebenen Bedeutungen hat, in Gegenwart von Wasserstoff in flüssiger Phase, erhalten kann, wenn man die Umsetzung mit einem wenigsten einfachen molaren Überschuß des Amins an einem im Reaktor fest angeordneten oder ständig darin befindlichen Katalysator, der als wesentliche aktive Bestandteile Kupfer und Chrom enthält, bei einem Wasserstoffdruck über 5 bar und Temperaturen von 150 bis 250°C kontinuierlich vornimmt, die Umsetzungsprodukte aus dem Reaktor kontinuierlich entfernt, das bei der Reaktion entstandene Wasser aus dem Reaktionsgemisch abdestilliert und nach Abtrennung der Amine der Formel I das nicht umgesetzte Amin und das teilumgesetzte Diol in den Reaktor zurückführt.

Zur Durchführung des Verfahrens kann man von reinen Alkan- oder Oxalkandiolen z.B. Butandiol-1.4, Pentandiol-1.5 oder Hexandiol-1.6 oder Diglykol, Diäthanolamin und ähnlichen, sich wie Diole verhaltenden Verbindungen ausgehen. Die Diole lassen sich auch als Mischungen einsetzen, beispielsweise als Gemisch der drei Diole, das durch Hydrierung von Rückstandsdicarbonsäuren der Adipinsäureherstellung leicht hergestellt werden kann. Die herzustellenden Amine können

in vielen Fällen in Form der entsprechend aufgebauten Mischungen verwendet werden.

Die umzusetzenden Amine verwendet man erfindungsgemäß in mindestens 2-fach molarer Menge, d.h. einfachem Überschuß, vorteilhaft jedoch in einem 2- bis 10-fachen Überschuß über die stöchiometrisch erforderliche Menge. Ein größerer Überschuß ist möglich, bringt jedoch keinen Vorteil.

Als Apparatur für die erfindungsgemäße Umsetzung dient im einfachsten Falle ein durchströmter Druckkessel, der den Katalysator enthält. Er sollte so ausgelegt sein, daß die Reaktanden eine mittlere Verweilzeit von 2 bis 4 Stunden haben können. Das Umsetzungsprodukt wird dann in der zweiten Stufe in einer Destillationskolonne fraktioniert destilliert. Einem Vorlauf von etwa 10 %, hautsächlich Wasser und Hydrogenolyseprodukten, folgen die Bis-dialkylaminoalkane mit Siedepunkten in der Regel um 70 bis 115°C/40 mbar. Höher siedende Anteile werden in den Reaktor zurückgeführt. Sie nehmen mit fortschreitender Umsetzungsdauer von 40 bis 50 auf 10 bis 30 Gew.% ab. Destillativ lassen sie sich trennen in ω-Dialkylaminoalkohole, ω,ω'-Alkandiole, d.h. Ausgangsmaterial und ca. 5 Gew.% höhersiedende Anteile. Bei diesen höhersiedenden Stoffen handelt es sich wahrscheinlich um Dimerisationsprodukte, die bei weiterer Umsetzung wieder zerfallen und sich erfahrungsgemäß nicht anreichern.

Bei Verwendung von Dimethylamin fällt dieses in einer gekühlten Vorlage an und wird nach Entfernung des Wassers zurückgepumpt. Die Wasserfraktion hat vom Dimethylamin ebenso wie vom nächstsiedenden ω,ω'-Bis-dimethylaminobutan (Sp. 170°C) einen großen Siedepunktabstand, so daß auch bei Kolonnen mit geringem Trennvermögen eine sehr selektive destillative Abtrennung des Wassers möglich ist.

Besonders vorteilhaft führt man die Umsetzung in einem druckfesten Röhrenreaktor aus. Der Katalysator ist darin fest, z.B. in stückiger Form angeordnet. Aus dem Reaktionsaustrag wird Wasser entweder chargenweise abdestilliert, oder man führt den Produktstrom im Kreis über eine Kolonne in der Wasser kontinuierlich abgetrennt wird.

Die für das erfindungsgemäße Verfahren geeigneten Katalysatoren sind an sich bekannt. Es handelt sich um Hydrierkatalysatoren, wie sie z.B. in der Zusammenfassung von Baiker und Richarz l.c. aufgeführt sind und als sogenannte Adkins--Katalysatoren, d.h. Kupfer und Chrom enthaltende Katalysatoren, die in nicht reduziertem Zustand Kupferoxid und Kupferchromit enthalten. In untergeordneter Menge, z.B. bis 5 % bezogen auf die übrigen Metalle, enthalten sie in der Regel noch Mangan, Zink oder insbesondere Barium. Es können trägerfreie Katalysatoren oder auch Trägerkatalysatoren verwendet werden.

Als Träger kommen geformte Körper wie Splitt, Strangpresslinge, Kugeln oder Ringe aus Bims, Magnesiumsilikat, Tonerde insbesondere γ-Aluminiumoxid oder Kieselsäure in Betracht.

Zur Herstellung der Katalysatoren geht man in der Regel so vor, daß man das Trägermaterial mit Lösungen von Salzen der beteiligten Metalle tränkt und im Wasserstoffstrom eine Reduktion zum metallischen Kupfer bzw. zur niedrigstwertigen Kupfer-Chromverbindung, die unter diesen Bedingungen beständig ist, vornimmt.

Eine andere Herstellungsmethode besteht darin, die Hydroxide der aktiven Metalle ggf. zusammen mit Aluminiumhydroxid auszufällen und den Katalysator durch anschließendes Kalzinieren bei 300 bis 500°C und Reduktion zu formieren.

Die fertigen Katalysatoren enthalten die genannten aktiven Metalle in Mengen von 0,5 bis 100, vorzugsweise 10 bis 100 Gew.%, bezogen auf den gesamten Katalysator.

Die erfindungsgemäß zu verwendenden Katalysatoren werden vorzugsweise in Form von Strangpresslingen oder Splitt angewendet, wobei die Katalysatoren Teilchengrößen von 2 bis 8, vorzugsweise 3 bis 5 mm aufweisen.

Beispiel 1

a) Ein Reaktionsrohr von 30 mm lichter Weite und 1 m Länge wird mit 600 ml eines Katalysators gefüllt, der 25 % Kupferoxid und 1 % Chromoxid auf Kieselsäuresplitt in Stücken von 4 bis 8 mm enthält. Der Katalysator wird mit einem Gemisch von Wasserstoff/Stickstoff zunächst bei 200 bar und 100 bis 200°C, dann mit reinem Wasserstoff bei 200 bar und 200°C reduziert und das entstandene Wasser anschließend entfernt.

Nachdem ein Druck von 40 bar Wasserstoff und eine Temperatur von 210°C im Reaktor eingestellt ist werden über zwei Dosierpumpen stündlich 200 ml geschmolzenes Hexandiol-1.6 und 350 ml Dimethylamin von unten nach oben durch den Reaktor gepumpt.

Der Reaktionsaustrag wird destillativ aufgearbeitet. Man erhält 30 Gew.% Dimethylamin/Wasser-Gemisch; 30 Gew.% N,N'-Tetramethyldiaminohexan-1.6 vom Sp. 110°C/40 mbar; 20 Gew.% N,N-Dimethyl-6-aminohexanol vom Sp. 135°C/40 mbar; 15 Gew.% Hexandiol-1.6 vom Sp. 162°C/40 mbar und 5 Gew.% Höhersiedende.

b) Die gemäß a) erhaltenen Fraktionen von Dimethylaminohexanol und Hexandiol werden vereinigt, 20 Gew.% Hexandiol-1.6, bezogen auf die Menge des Gemisches zugesetzt, und die Mischung unter Rückführung des vom Wasser befreiten Dimethylamin/Wasser-Gemisch und Ergänzung der im ersten Durchgang verbrauchten Dimethylaminmenge wie in a) beschrieben erneut durch das Reaktionsrohr geleitet.

Die destillative Aufarbeitung des Reaktionsaustrages liefert nun 30 Gew.% Dimethylamin/Wasser-Gemisch; 50 Gew.% N,N'-Tetramethyldiaminohexan-1.6; 10 Gew.% N,N-Dimethyl-6-aminohexanol und 10 Gew.% Hexandiol.

Beispiel 2

a) Ein Reaktionsrohr von 1 m Länge wird mit 600 ml des in Beispiel 1 beschriebenen Katalysators gefüllt. Durch Behandlung bei 250°C im Wasserstoffstrom, zuerst bei Normaldruck, dann bei 250 bar wird daraus der aktive Kontakt erhalten.

Nachdem die Temperatur im Reaktor auf 200°C und der Druck auf 250 bar mit Wasserstoff eingestellt ist werden über zwei Pumpen stündlich 200 ml Butandiol-1.4 und 350 ml Dimethylamin am unteren Ende des Reaktionsrohrs eingeleitet.

Nicht umgesetztes Dimethylamin und Wasserstoff werden am Kopf des Reaktors abgezogen und über einen Verdichter in den Reaktionskreis zurückgeführt. Der Reaktoraustrag wird destillativ aufgearbeitet und enthält 25 Gew.% Dimethylamin/Wasser; 25 Gew.% N,N'-Tetramethyldiaminobutan-1.4 vom Sp. 74°C/40 mbar; 25 Gew.%

N,N-Dimethyl-6-aminobutanol vom Sp. 100°C/40 mbar und 35 Gew.% Butandiol.

b) Die gemäß a) erhaltenen Destillate von N,N-Dimethylaminobutanol und Butandiol werden mit reinem Butandiol wieder auf die ursprünglich eingesetzte Menge gebracht und unter den Reaktionsbedingungen von a) zusammen mit frisch zugesetztem Dimethylamin erneut durch das Reaktionsrohr geleitet.

Der Reaktoraustrag enthält nun neben wäßrigem Dimethylamin 55 Gew.% Tetramethyldiaminobutan, 10 Gew.% Dimethylaminobutanol und 5 Gew.% Butandiol.

Beispiel 3

In einem Reaktionsrohr wie es in Beispiel 1 und 2 beschrieben ist, werden bei 250 bar Wasserstoffdruck und 220°C stündlich 200 ml Diglykol und 350 ml Dimethylamin umgesetzt.

Der Reaktionsaustrag enthält neben Dimethylamin-Wasser 35 Gew.% N,N'-Tetramethyl-bis(aminoäthyl)-äther vom Sp. 198°C, 20 Gew.% N,N-Dimethylaminoäthyl-glkyoläther und 25 Gew.% Diglykol. Die Rückführung von Ausgangsmaterial und Dimethylaminoäthyl-glykoläther erhöht den Anteil von Tetramethyl-bis(aminoäthyl)-äther im Reaktionsprodukt auf 60 Gew.%.

Patentanspruch

Verfahren zur Herstellung von Aminen der Formel I

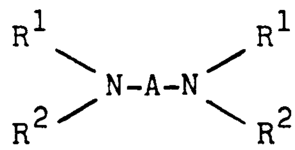

I,

in der A einen gegebenenfalls durch Sauerstoff- oder Stickstoffatome unterbrochenen Alkylenrest mit 4 bis 10 Kohlenstoffatomen und $R^1$ und $R^2$ Alkylreste, die hydrierbeständige Substituenten tragen können, bedeuten, oder die zusammen einen heterocyclischen 5- oder 6-gliedrigen Ring bilden, durch Umsetzung eines Diols der Formel II

$$HOCH_2-A-CH_2OH \qquad II,$$

mit einem Amin 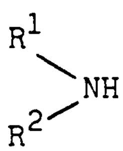, wobei A, $R^1$ und $R^2$ die oben angegebenen Bedeutungen hat, in Gegenwart von Wasserstoff in flüssiger Phase, dadurch gekennzeichnet, daß man die Umsetzung mit einem wenigsten einfachen molaren Überschuß des Amins an einem im Reaktor fest angeordneten oder ständig darin befindlichen Katalysator, der als wesentliche aktive Bestandteile Kupfer und Chrom enthält, bei einem Wasserstoffdruck über 5 bar und Temperaturen von 150 bis 250°C kontinuierlich vornimmt, die Umsetzungsprodukte aus dem Reaktor kontinuierlich entfernt, das bei der Reaktion entstandene Wasser aus dem Reaktionsgemisch abdestilliert und nach Abtrennung der Amine der Formel I das nicht umgesetzte Amin und das teilumgesetzte Diol in den Reaktor zurückführt.

Europäisches Patentamt

# EUROPÄISCHER RECHERCHENBERICHT

0006454

Nummer der Anmeldung
79101620.

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | US-A-3 223 734 ( FALLSTAD et al) * Beispiele V und IX * | 1 |
| | - - | |
| D | DE-A-2 535 073 (HOECHST) * Seite 4 letzter Absatz, Seite 5 1.Absatz * | 1 |
| | - - | |
| D | HOUBEN-WEYL "Methoden der organischen Chemie" 4. Auflage., Band 11/1 "Stickstoffverbindungen II" 1957, GEORG THIEME VERLAG, Stuttgart, Seiten 126 bis 134 * Tabelle 18 (2.Fortsetzung) * | 1 |
| | - - | |
| D | INDUSTRIAL & ENGINEERING CHEMISTRY, PROD.RES.DEV., Band 16, No.3, 1977 Washington, A.BAIKER und W.RICHARZ "Catalytic Amination of Long Chain Aliphatic Alcohols" Seiten 261 bis 266 * Seiten 263 bis 266 * | 1 |
| | FR-A-2 351 088 (BASF) * Beispiel 12 * | 1 |
| | - - | |
| | DE-A-2 749 066 (SHELL) * Beispiel 2 * | 1 |
| | - - | |
| A | DE-A-2 749 064 (SHELL) * Beispiel 2 * | 1 |
| | GB-A-1 077 949 (GULF RESEARCH & DEVELOPMENT) * Tabelle III * | 1 |
| A | GB-A-1 027 508 (BASF) * Beispiel 6 * | 1 |

KLASSIFIKATION DER ANMELDUNG (Int.Cl.²)

C 07 C 87/14
C 07 C 85/06
C 07 C 93/04

RECHERCHIERTE SACHGEBIETE (Int. Cl.²)

B 01 J 23/86
C 07 C 87/14
C 07 C 85/06
C 07 C 93/04
C 07 C 87/00
C 07 C 87/02
C 07 C 87/04
C 07 C 87/06
C 07 C 93/00
C 07 C 93/02
B 01 J 23/84

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Wien | 13.August 1979 | KÖRBER |